# EUROPEAN PATENT APPLICATION

(11) **EP 1 164 531 A2**
(43) Date of publication of application: **19.12.2001**
(21) Application number: 01305179.2
(22) Date of filing: 14.06.2001
(51) Int. Cl.: G06F 19/00

(54) **Patient healthcare system**

(30) Priority: 16.06.2000 US 595088
(71) Applicant: Pfizer Products Inc., Groton, Connecticut 06340 (US)
(72) Inventor: Babamoto, Kenneth Susumu, Palos Verdes Estates, California 90274 (US); Shamsian, Bahram, Los Angeles, California 90025 (US); Steintmetz, Michael Alan, West Hills, California 91304 (US)
(74) Representative: Rees, Alexander Ellison

(57) **Abstract**

The present invention is an automated disease management system designed to assist healthcare providers in the care management of patients with one or more health conditions. More particularly, this invention relates to a system and method for assisting healthcare providers in developing and monitoring the implementation of patient care plans.

## Description

### BACKGROUND OF THE INVENTION

Managing the healthcare process is a complex and expensive area of patient care. Traditionally the health concerns of patients are presented to a healthcare provider who in turn performs a diagnosis, therapeutic selection, resource selection, treatment regime and follow-up visits. This normal course of addressing the health concerns of patients can be further broadened to manage the healthcare of a patient by assisting patients in identifying various health concerns and conditions and planning for immediate and long-term actions in order to assist in managing them, particularly those which may be chronic or eventually curable. An emphasis has also been placed on preventive medicine and wellness in response to increasing costs of healthcare. The health concerns of patients now encompass preventive medicine and wellness.

Prior methods for managing the healthcare of patients included manual data entry systems in which data were entered into paper files of patients which were individually studied to render individually appropriate care plans or to collect information regarding general areas of care in order to generate substantive statistical information. It is self-evident that such methods of developing individual or general care plans for patients were highly labor-intensive, inefficient, time-consuming and ineffective.

More recently, as efficiency became a concern, attempts have been made to develop and utilize standard patient questionnaire forms, descriptions of conditions and treatment and other standardized information gathering forms in order to collect and study healthcare data. Newer systems integrate and automate the analysis of healthcare data, but they are mostly limited to financial data for accounting and administrative purposes.

Also known in the art are comprehensive systems and methods of managing patient scheduling, insurance, clinical examination, billing, entering and displaying data to a physician, updating patient data, recording diagnosis and prescription information. These systems allow for concurrent recording of examination and diagnoses notes in a database during patient examination. One such system and method is disclosed in U.S. Patent No. 5,772,585. Another such system, disclosed in U.S. Patent No. 5,953,704, collects information on individuals having a health concern at any stage, guides the user to a system-selected treatment based on the information collected, and compares an actual or proposed treatment with the system-selected treatment.

However, such systems as these known in the art do not assist healthcare managers and patients in developing specialized healthcare plans that are individually tailored for a particular patient's history, symptoms, and diagnoses for one or more than one health conditions. Accordingly, there is a need for an automated system that assists healthcare providers and patients in achieving long-term and short-term patient healthcare goals such as weight loss plans, exercise plans, alcoholism and smoking programs and other forms of health improvement actions on an individual-patient basis.

### SUMMARY OF THE INVENTION

The present invention is an automated, disease management system designed to assist healthcare providers in the care management of patients with any of several disease or other health-related conditions. The system provides for the efficient capturing of patient information that permits information to be processed in connection with a plurality of clinical modules containing data on various medical conditions to produce treatment recommendations, patient and task tracking facilities and outcomes reporting, all from a single integrated application.

The system of the present invention also supports a wide range of case management functions, with tools such as: patient-specific task lists, reminders, notes, tracking of patient-specific clinical history and telephone contacts and even the automated generation of report and reminder correspondence. The system can be utilized by a variety of healthcare providers such as physicians, physician assistants, nurses, administrators, etc. who each contribute to the development of a specialized care plan for patients based on their prior history, diagnosis, clinical notes, treatment, medical staff assessments, diagnosis, observations, therapy sessions, follow-up visits, medication and any other factors that may affect the patient's medical conditions. The system is expansive in that it allows for a large amount of information to be added to the database and allows access to an informative reference guide in assisting healthcare providers in understanding more about a particular health condition with which a patient is diagnosed, including its various associated symptoms, and various methods of coping with the condition, ways to cure the condition and various care plans that could be established for a particular patient depending on the gravity of the particular condition and the patient's age, overall health, other conditions she may be diagnosed with, and other factors.

The system allows the user to enroll patients having one or more health conditions, i.e., alcoholism, asthma, high cholesterol, compliance, diabetes, high blood pressure, smoking-related conditions and many others, into an array of case management programs. In addition, associated with such case management programs are appropriate actions that can be selected for each selectable care plan category and related tasks that can be scheduled for each patient enrolled in a care plan program. With the selection of a main care plan category, all available actions specific to the selected healthcare category appear on the screen for selection by the healthcare provider. These include guidelines, suggested interventions and other action items that are suited to assist in managing the patient's particular conditions. In addition, the system contains the ability to consider the interactions among health risks, medications, age of patient, enrollment into particular care plans, selected action items and other factors.

The system of the present invention uses the metaphor of a Master Cabinet with several file drawers, each drawer containing folders which contain data and functions related to patients, providers and tasks. Folders for each patient contain tabs such as the Care Plan tab associated with development of a care plan and related action items for each patient. Associated with selection of each action item selected is a reference tool that generates the corresponding section of its reference drawer resource to assist the care manager or patients in understanding and being more informed about the particular area of care.

### BRIEF DESCRIPTION OF THE DRAWINGS

FIG. 1 is a flow chart of one embodiment of the system for formulating a healthcare management plan for a patient.
FIG. 2 shows a Care Plan tab screen and its features.
FIG. 2a shows a Care Plan tab screen with the remainder of its fields visible (after having scrolled the window to the right using the right-arrow button).
FIG. 3 shows a New Care Plan Actions screen identifying various available care plan categories and actions items.
FIG. 4 shows a New Care Plan Actions screen showing how one can select between care plan categories or program/contacts.
FIG. 5 is a New Care Plan Actions screen identifying various action items for a selected program/contacts.
FIG. 6 is a New Care Plan Actions screen showing the drop-down list for the "Assign To:" field.
FIG. 7 shows a New Care Plan Actions screen showing the selection of an item in the New Care Plan Actions list in preparation for the new, edit and remove button screens.
FIG. 8 is a view of the New Care Plan Action box after selecting the new button as shown in Fig. 7.
FIG. 9 is a view of the Edit Care Plan Action box after selecting the edit button as shown in Fig. 7.
FIG. 10 is a view of the Patients Drawer.
FIG. 11 is a view of the Providers Drawer.
FIG. 12 is a view of the Tasks Drawer.
FIG. 13 is a view of the Demographics tab folder found within the Patients Drawer as shown in Figures 2 and 10.
FIG. 14 is a view of the Enrollment tab folder found within the Patients Drawer as shown in Figures 2 and 10.

### DETAILED DESCRIPTION

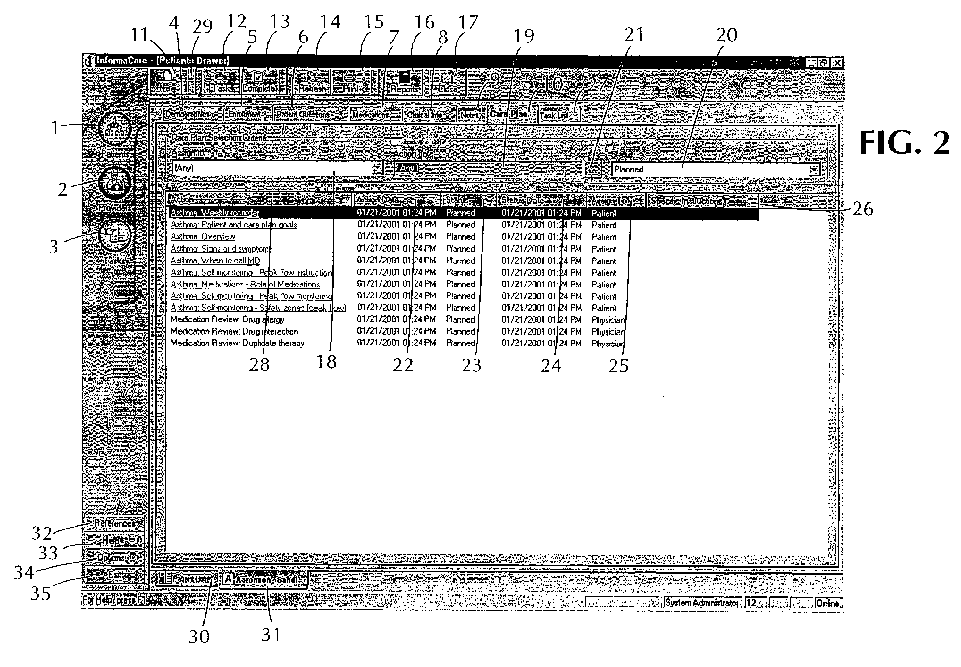

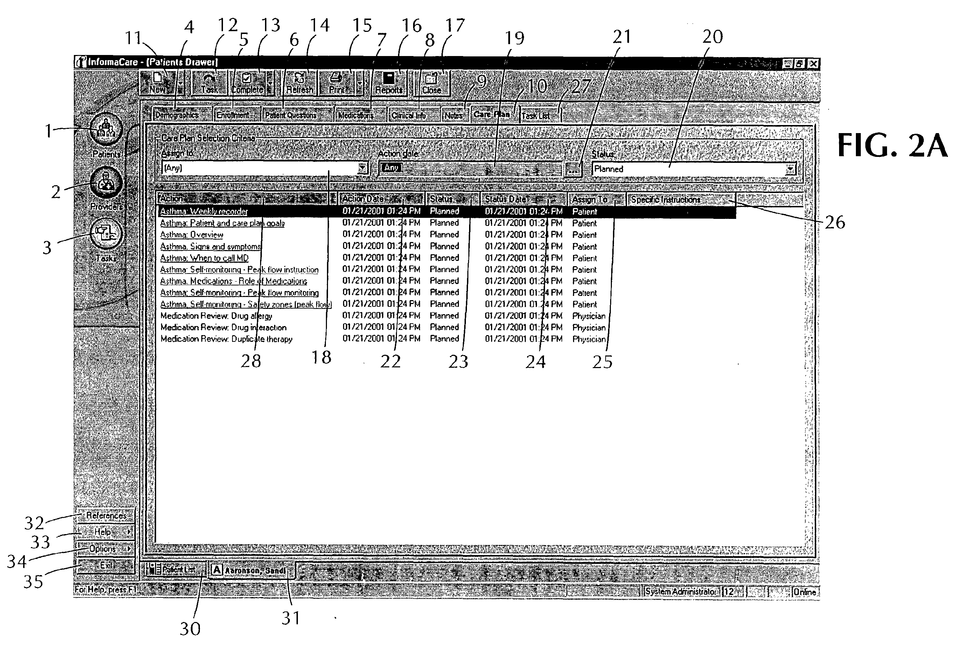

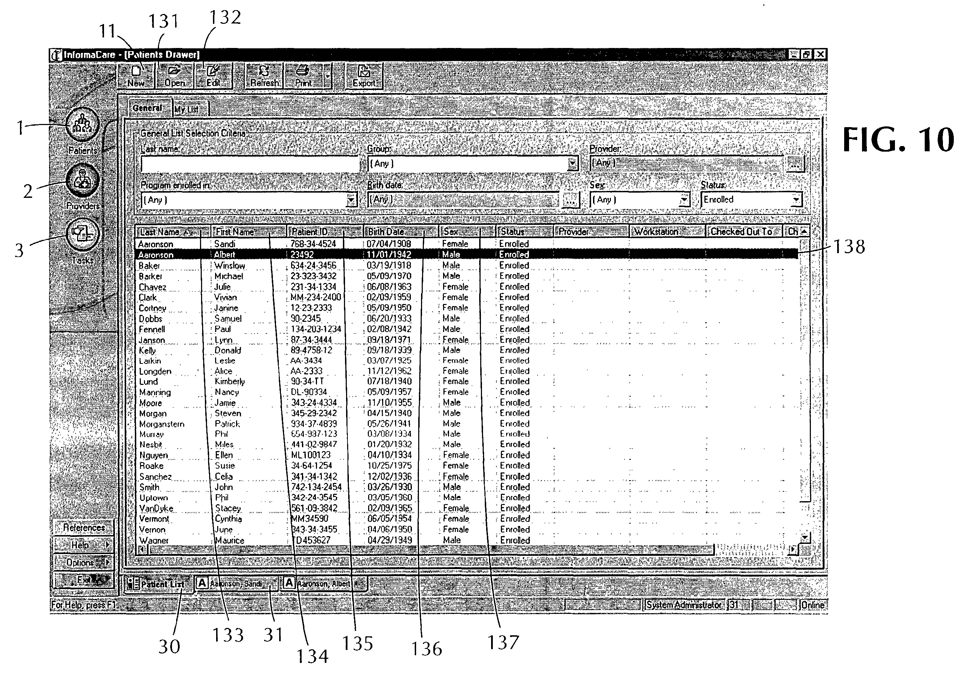

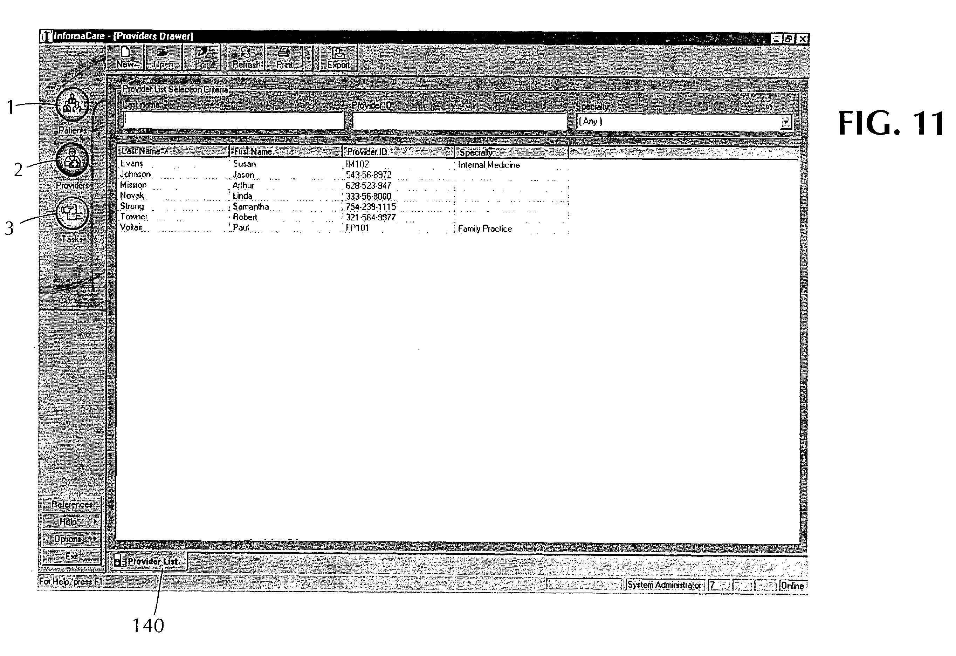

From the user's perspective, the system of the present invention acts as a "Master Cabinet" with several file drawers as shown in Figures 10-12 each drawer containing "folders" which in turn contain data and functions related to patients (1), providers (2) and tasks (3). Folders for each patient contain tabs within the patients drawer (1) as shown in Figure 2, for example, for Demographics (4), Enrollment (5), Patient Questions (6), Medications (7), Clinical Information (8), Notes (9), Care Plan (10), and Task List (27). For providers, as shown in Fig. 11, the overall "drawer" view shows, as an index to the contents of the drawer, a list of providers by name, provider identification number and specialty. Within the drawer, each provider "folder" contains more complete information about each provider.

After a user (which can be a physician, nurse, health care provider, health care manager, administrator, etc.) has signed on to the program, she can collect from its database, among other information, clinical data such as lab results, physical exam and patient questions for various patients. A user may also enroll patients into various case management programs through the system commands. The user may also view historical data and activities for a patient, view and print provider reports, create tasks associated with patient care, sort and edit task information such as patient visit scheduling, patient details, patient phone number and best time to contact, provider name, task status and priority.

The user can use all the data previously entered for a patient such as patient questions, medications, clinical information and notes tabs to assist in formulating an individual and unique care plan for each particular patient. The user selects appropriate action items for each category and uses the corresponding data found in the other "drawers" of the "cabinet" to assist with selecting all care plan action items for a particular patient.

The system of the present invention is thus a resource for extensive patient information that is used to formulate a healthcare program unique to each patient, that assists both health care providers and patients in managing a particular patient's condition(s).

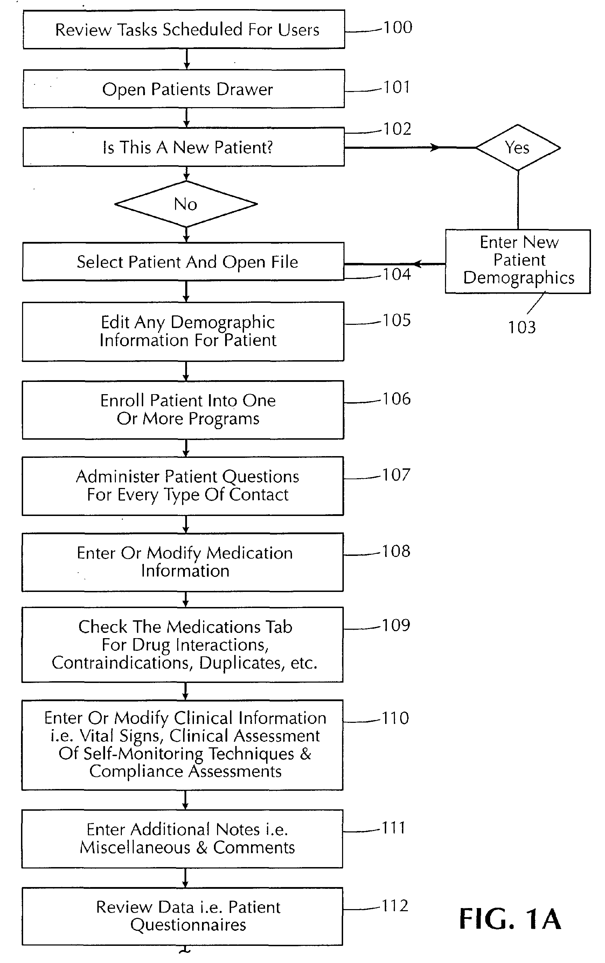

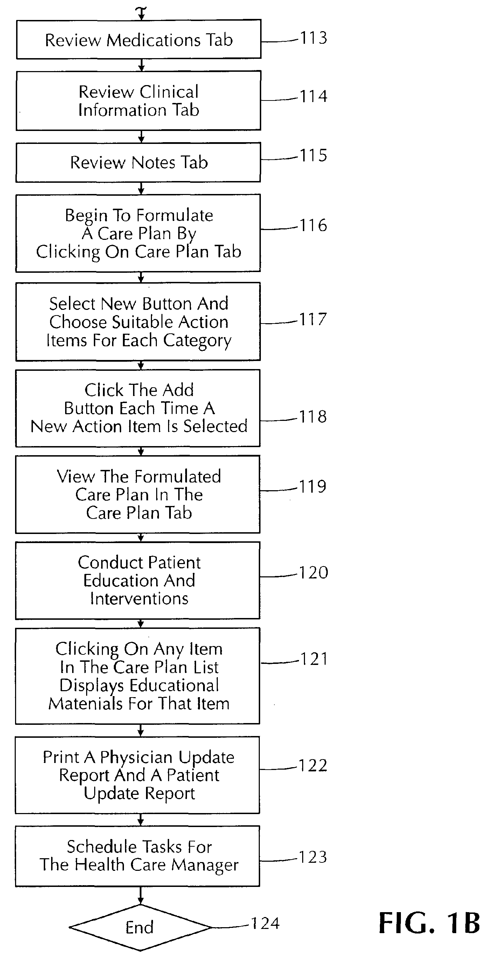

Figure 1 is a flow chart of one embodiment of the invention showing the process of developing a suitable healthcare plan for a particular patient. In this process, the following sequence of steps would generally occur during or after patient contact, although the actual sequence may vary depending on one's preferences, the situation and the appropriateness of particular actions for individual patient circumstances.

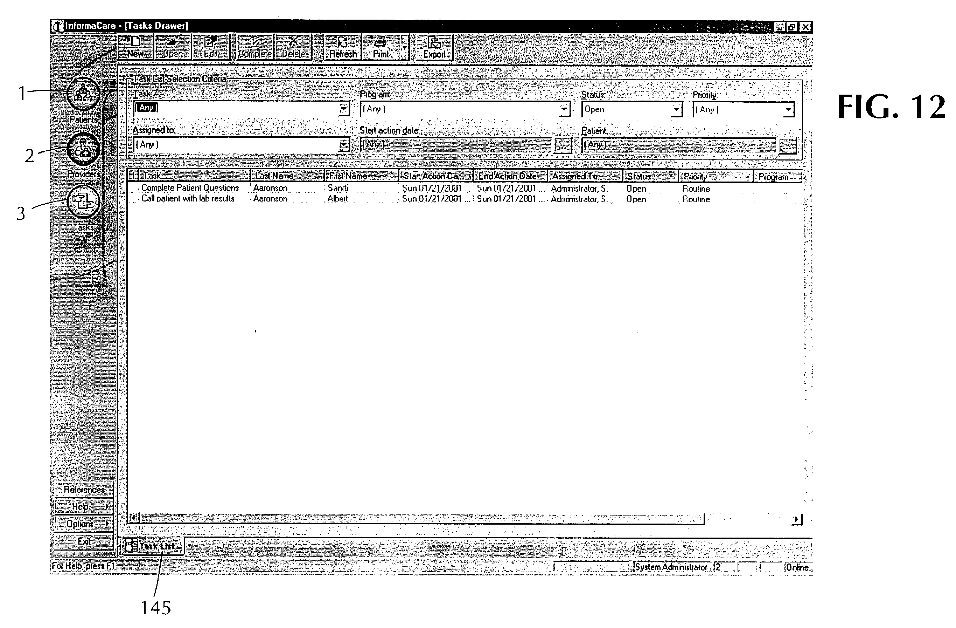

The system user would first review any tasks scheduled (100) for herself that day as shown in Figure 1, by clicking on the Tasks button (3) as shown in figure 2. (It should be understood that, while this description uses the term "user" of the system, in fact the user could be a physician, physician assistant, nurse, administrator, etc.) The tasks button (3) opens the Tasks Drawer and enables the user to view what tasks have been scheduled. Figure 12 is an example of what a user may find in the Tasks Drawer.

If a patient visit was a scheduled task, the user would then open the patients' drawer (101) as shown in Figure 1, by clicking on the Patients Drawer button (1) as shown in Figure 2. If the visit is the patient's first contact with the healthcare provider, the user would enter new patient demographics (103) as shown in Figure 1, by clicking the New button (11) as shown in Figure 2 and entering personal data for the patient. In the preferred embodiment, the only fields that must contain data are: First Name (134), Last Name (133), Birth Date (136), Sex (137) and Patient ID (135) as shown in Figure 10. If the patient is not a new one, the user would proceed to select that patient's file (104) as shown in Figure 1, by first clicking on the patient's name from the patient list (30) file folder as shown in Figure 10 (which lists every patient alphabetically by last name) and then opening the highlighted patient's file folder (31) (Sandi Aronson (138) is highlighted as shown in Figure 10) as shown in Figures 2 and 10. The resulting screen seen after opening the patient's folder is shown in Figure 2.

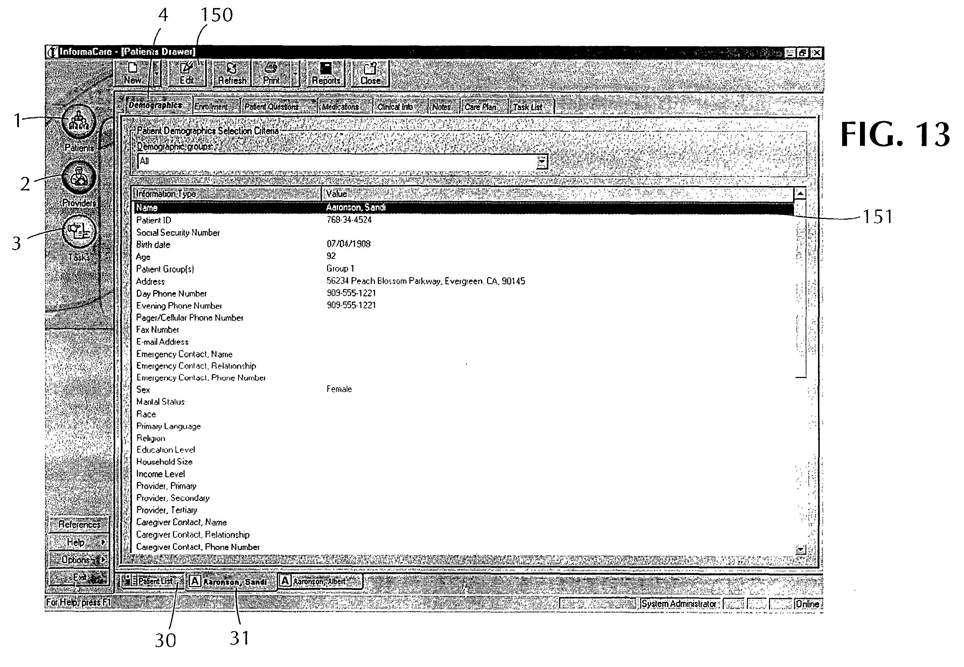

From within the patients drawer (1) as shown in Figures 10, the user would click within the row containing the name of the patient (138) she wishes to select and click the open button (131) to access all information for that particular patient. In the preferred embodiment, the information is organized into eight tabs or sections within the folder with tabs as shown in Figure 2, for demographics (4), enrollment (5), patient questions (6), medications (7), clinical information (8), notes (9), care plan (10) and task list (27). The user then may edit any demographic information (105) as shown in Figure 1 for the selected patient, by clicking the edit button (132) while in the patient list folder (30) as shown in Figure 10 or while in the demographics tab (4) of the patient's record folder (31) by clicking on the edit button (150) as shown in Figure 13.

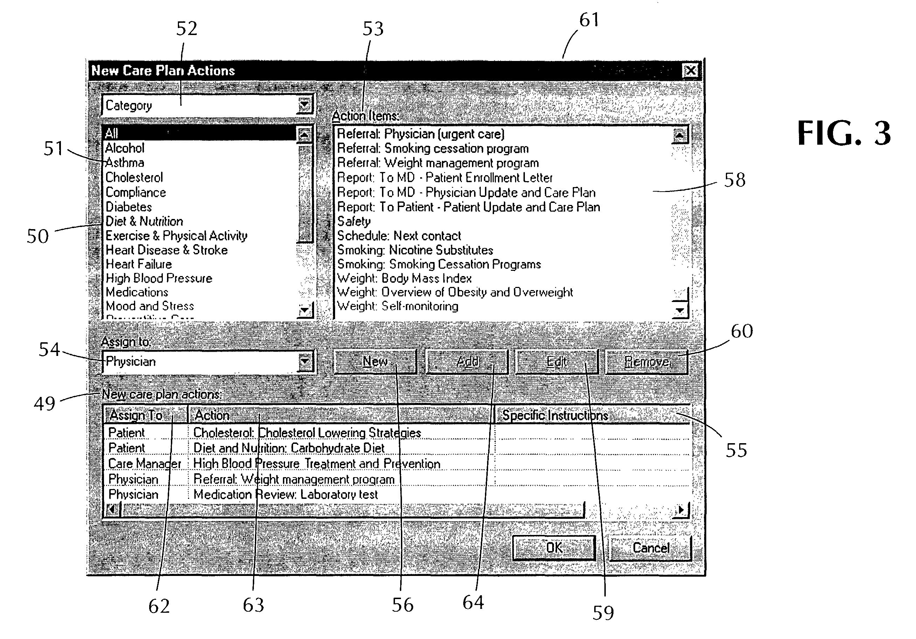

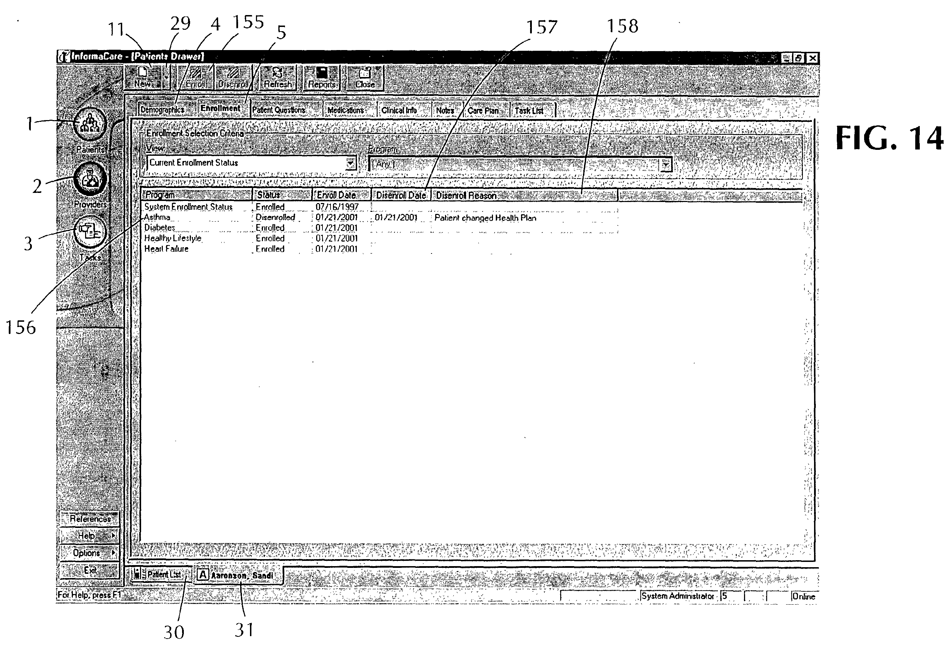

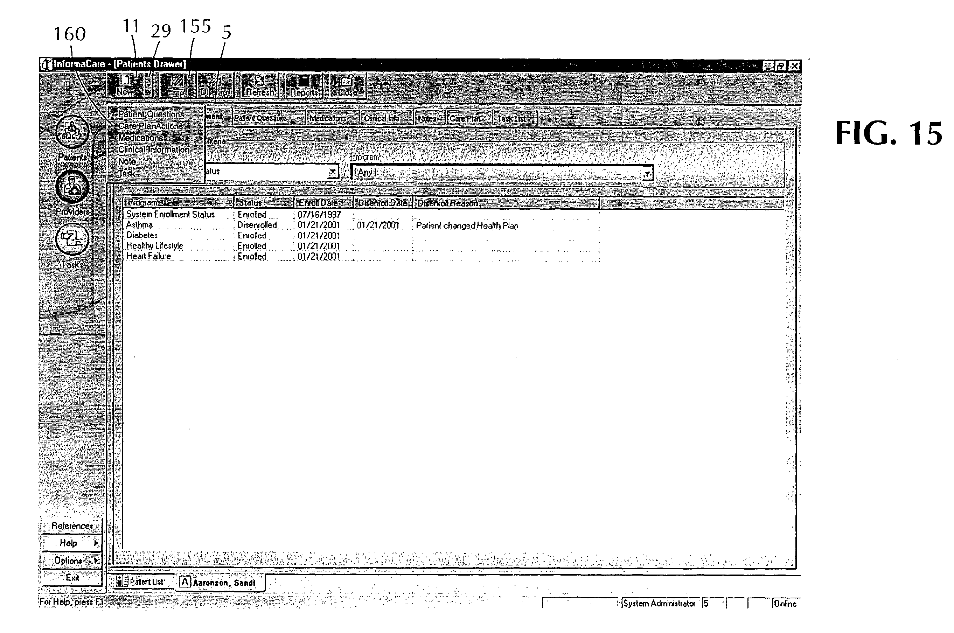

The patient should be enrolled into one or more programs (106) as shown in Figure 1, before proceeding with other procedures such as administering questionnaires, entering medications, and formulating a care plan, and a patient may be enrolled in multiple programs. To enroll a patient, the user opens the patient record folder (31), clicks the enrollment tab (5) as shown in Figure 14, to view the patient's enrollment history, then clicks on the new button (11) and then clicks on the new care plan actions option as shown in Figure 15, by first clicking on the drop-down arrow (29) located next to the new button (11) as shown in Figure 14 and selecting the care plans action option (160) as shown in Figure 15. The user next selects the name of the program (50) in which she wants the patient enrolled from the New Care Plan actions screen as shown in Figure 3. The user may also enroll a patient into a program for which she is currently disenrolled as shown in the patient's enrollment history in Figure 14 under the Disenroll Date (157) or Disenroll Reason (158) column. The user simply selects the disenrolled program for e.g. Asthma Program (156) and then clicks the enroll button (155) to enroll the patient into the particular program as shown in Figure 14.

Next the user asks the patient prescribed questions for each type of contact, *i.e*., initial or follow-up (107) as shown in Figure 1. Depending on the type of contact, initial or follow-up, within each program there are suggested patient questionnaires which can be accessed by clicking on the patient questions tab (6) in the patient record folder as shown in figure 2 and then clicking the new button (11). The appropriate radio buttons and checkboxes can be clicked to select the contact types and question sets of the user's choice. The user has the option of printing, viewing or both the Patient Reported Information report upon completion of the questionnaire.

The user may next proceed to enter or modify medication information (108) as shown in Figure 1. Medication information will usually come from the patient or the patient's healthcare provider. To enter or modify medication information, the user clicks the Medications tab (7) as shown in Figure 2 in the Patient Record folder (31). The user next clicks the New button (11) to enter new medications or clicks an existing medication in the list and clicks the edit button to make changes. After the medications are entered, the user can reference the list in the Medications tab to check for drug interactions, contra-indications, duplicates, etc. The user may proceed to check the list of medications in the medications tab (7) for any drug interactions, contra-indications, duplicates, etc. (109) as shown in Figure 1, that a physician may have noted for the patient. The user may next enter or modify clinical information, *i*.*e*., vital signs, clinical assessment of self-monitoring techniques and compliance assessments (110) as shown in Figure 1. The user simply clicks the clinical information tab (8) as shown in Figure 2 in the patient record folder (31) and then clicks the new button (11) to enter new information or clicks the edit button to modify information that is highlighted in the list. The list provides a summary of patient data.

The user may next enter any additional notes (111) and comments for each patient as shown in Figure 1. To create a new note, the user may click the Notes tab (9) as shown in Figure 2 in the Patient Record folder (31) and then click the New button (11). To read or modify an existing note, the note should be highlighted in the list and the edit button clicked to make changes.

The user may review the data provided in patient questions (112), review medications (113), review clinical information (114), and review the notes tab (115) as shown in Figure 1. Finally, the user may begin to formulate a care plan by clicking on the care plan tab (116) in the patient record folder and then clicking on the new button (117) as shown in Figure 1.

After the user has reviewed the patient questions, medications, clinical info, and notes tabs, she is ready to formulate a care plan. Appropriate actions can be selected for each category selected by clicking the add button (64) as shown in Figures 3-7 each time a new action item is selected (118) as shown in Figure 1 and clicking the OK button once the user has completed her selection. Once the user has formulated the complete care plan for the patient, they can be viewed in the care plan tab (119) and the user can utilize the pooled information for a particular patient to conduct patient education and interventions (120) as shown in Figure 1.

When the user clicks the underlined items in the care plan list (121), education material materials for that item appear on the screen. For each action, the Complete button drop-down list (13) may be utilized to note the status of actions with either planned, pending, complete or delete action as shown in Figure 2. The actual status for each assigned action is found in the status (23) column as shown in Figure 2.

After the user has entered patient data and care plan information and the clinician administers the educational intervention, the user can print a physician update report for the patient's records or for the physician (122) as shown in Figure 1. The patient update report is useful for reviewing care plan items and viewing the status of clinical measurements and medication. To run a report, the user may click the Reports button (16) from any tab in the Patient Record folder (31) as shown in Figure 2. Finally, the user may schedule tasks to help manage future visits and reminders for the care manager (123) as shown in Figure 1. To create a new task, the user clicks the Task List tab (12) found in the Patient Record folder (31) and then clicks the New button (11) as shown in Figure 2. To view or modify an existing task, the user may click the Edit button when that task is highlighted in the list. At this point, the healthcare management process is complete for that particular patient visit (124) as shown in Figure 1.

The system consists of at least two overall configuration modes. A single user can operate the system in a standalone configuration. Alternatively, a workgroup configuration is available to enable more than one user to access the system from more than one workstation. The workgroup configuration allows all the users to share information about the patients, providers and tasks entered in the system. Such system would allow access through an online database with connection through a network or over a modem. In either case the hardware components are standard and well known to persons skilled in the personal computer art, including personal computers, associated displays and printers.

In one embodiment of the invention, the system as described in connection with FIG. 1, can be thought of as a file cabinet which contains three main drawers - patients (1), providers (2) and tasks (3). The user simply clicks the drawer button (1, 2, or 3) of her choice located on the left side panel of the screen as shown in Figure 2. The first folder in every drawer of the file cabinet contains a list of all the items in the drawer. For example, the Patients Drawer (1) has a patient list folder in which both a general tab and personal roster of patients for a particular user which may be accessed. The Providers Drawer has a Provider List folder and the tasks drawer has a Task List folder.

Each drawer contains a list folder which serves as an index to all the items in that drawer. Folder labels at the bottom of the screen indicate what folder are open and which one the user is currently using. For example, the first folder in the patients drawer is the patient list folder. If the user selects a patient from the list by double-clicking their name, a new folder is opened containing the details on the highlighted patient name. The folder label will display the patient's name. Labeled tabs appear along the top of some screens which are sub-folder within the open folder. The function buttons are at the top of the window. These are context-sensitive button bars which may change depending on what drawer, folder, or tab the user is using. The four buttons located on the bottom left hand corner of the window are referred to as global buttons which include references (32), help (33), options (34), exit (35) as shown in Figure 2. They are available regardless of which drawer or folder the user is located in. Clicking on the references (32) button displays the help topics references window which allows the user to either select a topic for further inquiry or to run a search for a word or topic the user is seeking further information regarding.

Figure 2 is a snapshot of the patients drawer that includes several tabs such as Demographics (4), Enrollment (5), Patient Questions (6), Medications (7), Clinical Information (8), Notes (9), Care Plan (10), and Task List (27). The Care Plan (10) tab is highlighted in this figure 2 since it has been selected. The Care Plan (10) tab contains all care plan action items defined for the selected patient., Sandi Aaronson, as shown on the bottom tab. The care plan action items are selected and entered into the system by the user. Each patient's record folder (31) will have access to her own care plan (10) tab. The individual care plan programs must be developed by the user to suit the particular patient's needs or patient requests which lends itself to being a patient-centric application. If the patient has any goals or improvements in their health or lifestyle, such endeavors can be set for the patient and monitored by the user in the care plan tab (10). All the various care plan action items previously associated with a particular overall care plan selected for this patient appears in the Action column (28). Care plan action items are organized into different categories such as Alcohol, Cholesterol, Exercise, High Blood Pressure, and Smoking. A user can select whether to view the action items for the current day or select a day of her choice. The action date (22), status date (24), status (21) and the identity of the entity to whom the action is assigned ("Assign To") (25), and specific instructions (26) as shown in Figure 2a for that particular action are all available in the Care Plan screen. The "Assign To:" field (25) allows the user to see action items defined only for a Patient, Care Manager or Physician. The "Action date:" field (22) allows the user to select a date on which the action item(s) took place. The "Status:" field (21) allows one to choose to see each care plan component or only the subset that are planned, pending, completed or, possibly, deleted items.

As shown in Figure 2, the system of the present invention acts as a "master cabinet" having several drawers. Each drawer contains data and functions related to various functional areas i.e. Patients (1), Providers (2) and Tasks (3). Each patient's folder selected after selection of patients drawer (1) includes a series of patient-centric tabs shown in Fig. 2, including demographics (4), enrollment (5), patient questions (6), medications (7), clinical information (8), notes (9), care plan (10) which has been selected in Figure 2, and task list (27).

The Care Plan tab (10) contains several function buttons including, as can be seen in Fig. 2: New (11), Task (12), Complete (13), Refresh (14), Print (15), Reports (16) and Close (17). The New button (11) opens the New Care Plan Actions dialog box as shown in Figure 3, for entering new action items based on the categories chosen and care plan developed by the user for a particular patient. Clicking the New (11) Button's drop-down arrow (29) as shown in Figure 2, will display a list of other types of activities such as creating a note or working with medications. The Task (12) button opens the New Task dialog box and enables the user to create a task from the selected action item. It is not available when a completed action item is highlighted. Selecting the Complete (13) button marks the selected event as complete. Selecting the Complete button's drop-down arrow displays a list of other status options you can choose for the selected task such as Pend(ing), Complete, Delete. Once a task is marked as complete, the Complete button is no longer available. The Refresh (14) button refreshes the screen with any changes from the database. The Print (15) button prints the entire list. Clicking on the down-arrow button enables the user to go directly to Print Preview (to preview the list before printing). The Print Setup option allows the user to display a window where one can change or view the printer's default settings. The Reports (16) button enables one to display the Reports dialog box wherein various report forms can be generated depending on the user's preferences and goal at hand. The Close (17) button permits the user to close the current Patient Record folder and returns to the General Patient list tab (30) of the Patients Drawer or the most recently accessed Patient Record folder that is still open (31). The Care Plan tab (10) includes several selection criteria fields:
- Assign To (18) -- wherein all action items are shown automatically. This field is used to see action items defined only for a patient, care manager or physician.
- Action date (19) -- which allows one to select a date on which the action item(s) took place. The browse button "..." (21) can be used to select a date.
- The status button (20) -- an action item with any status shown automatically. This criteria is used to see only planned, pending, completed or deleted action items.
- The Care Plan tab (10) also consists of several column headings. The action (21) field displays the name of the action item.
- The action date (22) field displays the date and time the action item was started.
- The status (23) field displays either the planned, pending, completed or deleted field.
- The status date (24) field displays the date and time the status was last changed.
- The assign to (25) field displays patient, care manager or physician.
- The specific instructions (26) field displays any comment associated with this action item as shown in Figure 2a.

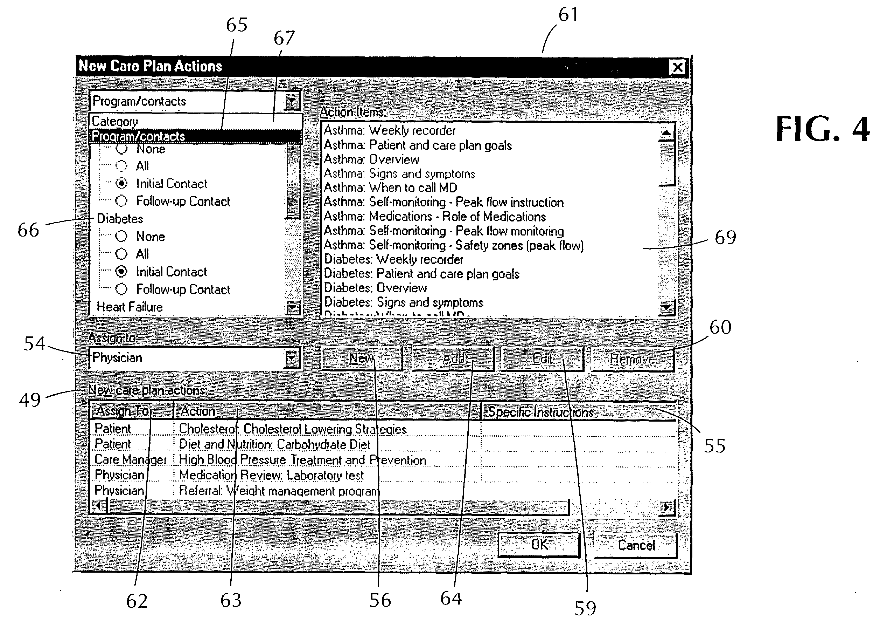

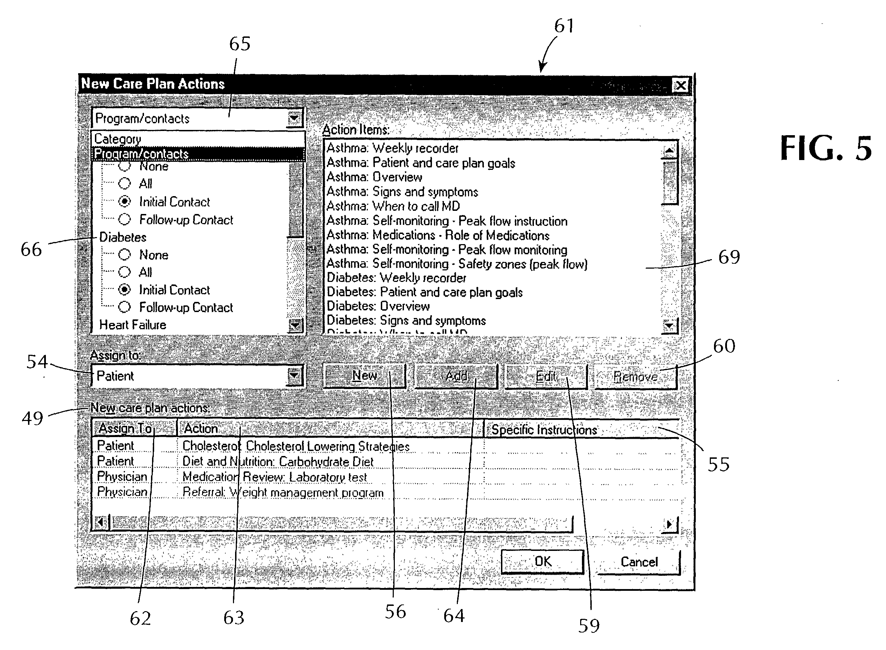

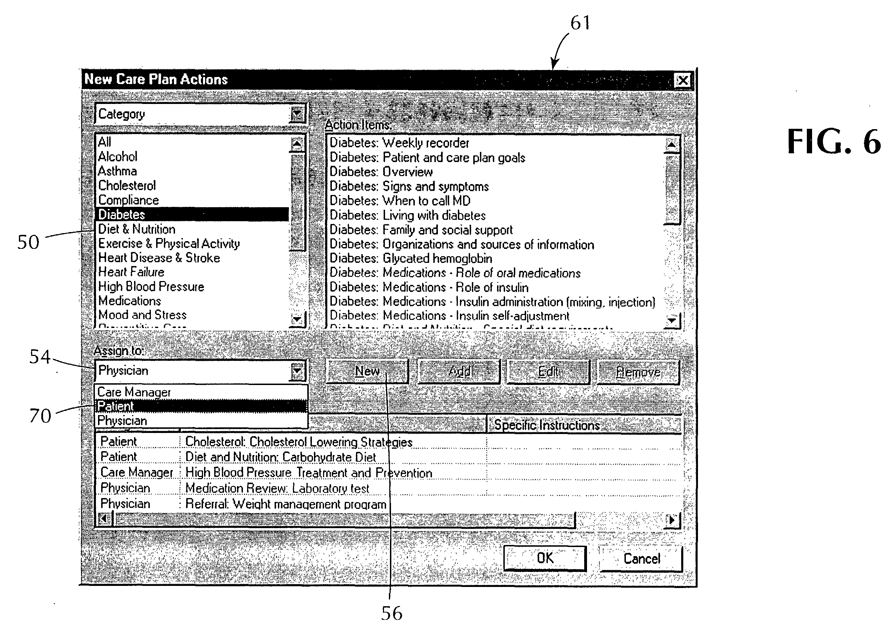

The New Care Plan Actions Dialog Box (61) as shown in Figure 3 can be accessed by clicking the New (11) button in the Care Plan (10) tab of the selected patient record folder (31) as shown in Figure 2. The user can select an action that is appropriate to the category or program/contacts of their choice as shown in Figure 3. The user may create new actions, select actions from the list, edit existing actions, or remove actions already selected. The Edit (59) and the Remove (60) buttons become enabled when an action is highlighted in the New care plan actions list (58). When category (52) is selected in the upper-left drop-down list in the New Care Plan Actions dialog (61) box as shown in Figure 4, the list box below it contains each available healthcare category (50) including "All" categories as shown in Figure 3. The user may either select the category list (52) or the "Program/contacts" (65) list as shown in Figure 4, from the category drop down list (50). As shown in Figure 5, the program/contacts (65) list contains contact types (66) such as none, all, initial contact, and follow-up contact for each available program (i.e. asthma, diabetes, heart failure, healthy lifestyle and others). If the category (67) field is selected in the upper-left drop-down list of the New Care Plan Actions dialog box (61), all available actions specific to the selected healthcare category (50) selected appear as shown in Figure 3. These can include guidelines, suggested interventions, and other action items for selection by the user. If Program/contacts (65) is selected, guidelines specific to the selected type of contact for the selected program (initial, follow-up, etc.) (66) are available in the action items (69) box as shown in Figure 5. As shown in Figure 6, the Assign To: (54) field permits the user to select either a care manager, patient or physician (70) to a particular new care plan action item selected. There are column headings that are displayed in the New Care Plan Actions (61) dialog box as shown in Figures 3-7: the Assign To (62) column heading shows to whom the action is assigned, the Action (63) column heading shows the name of the heading, and the Specific Instructions (55) column heading shows miscellaneous notes regarding the action. The following buttons appear in the New Care Plan Actions dialog box: the New (56) button is utilized to create a new action that does not already appear in the list. This essential tool permits a Care Manager or Physician to develop highly unique care plans for patients depending on various factors manifested in the patient which among other factors are diagnosis, clinical notes, lab results, personality traits, flexibility of the patient, mental and emotional condition or state, individual preferences and tendencies, and progress notes. The Add (64) button adds the highlighted action to the New care plan actions list (49). The Edit (59) button allows modification to the highlighted action in the New care plan actions list (49). The Remove (60) deletes the highlighted action from the New care plan actions list (49).

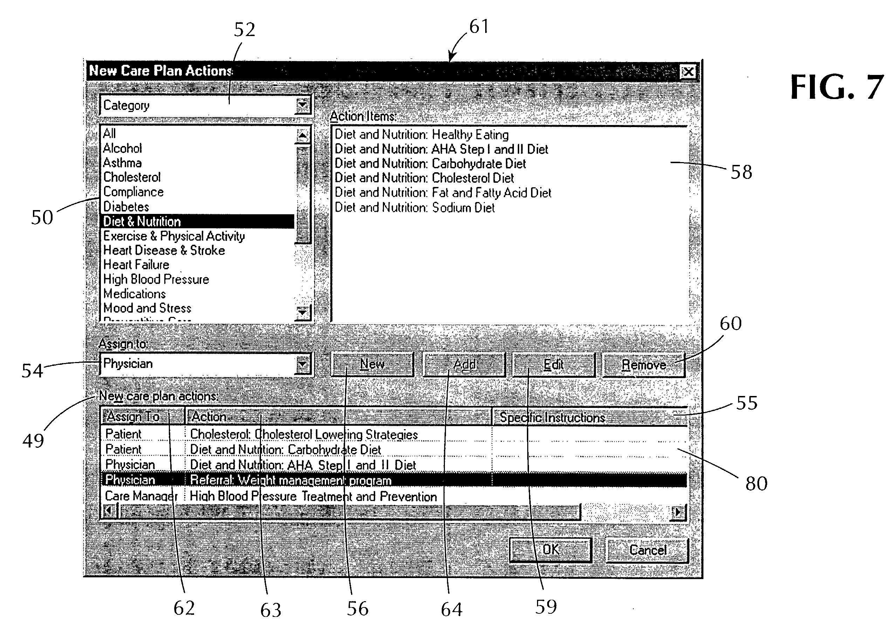

Figure 7 is a screen display of the New Care Plan Actions box (61) with a particular New care plan action selected i.e. the Diet and Nutrition: AHA Step I and II Diet (80). From this point, the healthcare manager may proceed to exit, add new action items, edit already added action items, remove action items or simply exit from the screen.

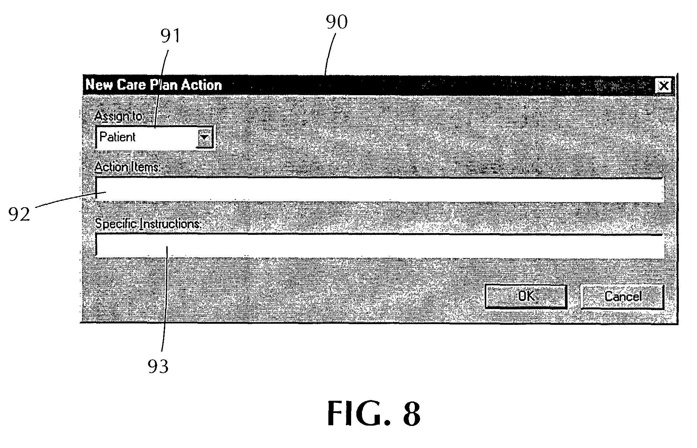

If the healthcare manager seeks to insert a new care plan action, the New Care Plan Action dialog box (90) as shown in Figure 8 appears by clicking the New button (56 ) in the New Care Plan Action dialog box (61) as shown in Figures 3-7. If an action is sought which does not appear in the New Care Plan Actions dialog box list (58), one can now be created by the user. As shown in Figure 8, New Care Plan action box (90) appears once the New button (56) has been selected as shown in Figures 3-7. The New Care Plan Action box (90) permits the healthcare manager to input action items and specific instructions for a particular patient that are not otherwise found among the existing selection of action items (58) as shown in Figure 7. The New Care Plan Actions box (90) permits the healthcare manager to develop the most suitable and unique management care plan for the particular patient whose needs may not be necessarily met by the already provided selection of action items (58). As shown in Figure 8, the assign to (91) field permits the action to be assigned to the patient, care manager or physician. The action items (92 ) field is a text field for entering the name of the care plan action that one wants to create. The specific instructions (93) field is a text field for entering individual notes regarding the care plan action selected for the particular patient.

If a healthcare provider wanted to enroll a patient into more or new care plan actions, the provider would simply select the new (11) icon which would generate a new menu and screen as shown in Figure 3. The New Care Plan Actions Screen (61) as shown in Figure 3 includes various categories of care plans. If, for example, the "Asthma" category (51) was selected, then the various corresponding "Action Items" would appear in the Action Items screen (52) particular to the Asthma category (51) and from which several actions could be selected for a particular category of care for a patient.

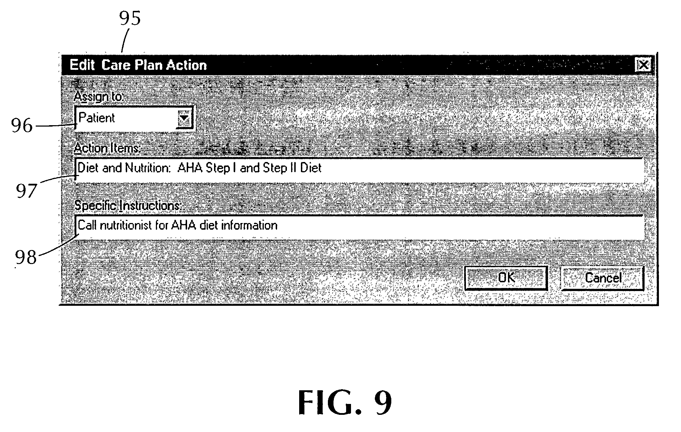

Figure 7 shows the screen from which the healthcare manager may proceed to edit a particular care plan action that was already selected i.e. the Diet and Nutrition: AHA Step I and II Diet (80). From this screen, the Edit button (59) can be selected. As shown in Figure 9, the Edit Care Plan Action box (95) appears after selecting the Edit button (59). The healthcare manager may now edit the Assign to (96), or the Specific Instructions (98) fields in accordance with a change in the patient's needs, progress or simply a change in the type of care plan action originally selected for the patient. The Action Item (97) can not be edited at this screen. The user at this point is editing the assign to (96) and specific instructions (98) field associated with the pre-selected action items (97).

It is intended that the foregoing detailed description be regarded as illustrative rather than limiting, and that it be understood that the following claims, including all equivalents, are intended to define the scope of this invention.

## Claims

1. A system for assisting a user in developing, administering and monitoring healthcare plans for patients having multiple conditions, said system permitting the viewing of data organized from a plurality of perspectives including at least the perspectives of patients, healthcare providers and tasks related to a patient's healthcare plan to be administered by the providers, the system comprising:
(a) a processor having associated input, memory and display means;
(b) the memory means being capable of retrievably storing data comprising (i) patient records having multiple fields of health-related data for each of a plurality of patients, (ii) healthcare plan data, (iii) patient demographics, (iv) information about a plurality of healthcare providers and (v) healthcare tasks relating to each patient and assigned to providers;
(c) the display means being capable of displaying at least
(i) a task view that presents a list of tasks scheduled for accomplishment by a provider during a selected time period;
(ii) a provider view that presents, at the user's option, either a list of providers and a first body of information about each provider or a second, more extensive, body of information about a particular one of said advisors; and
(iii) a patients view that presents, at the user's option, either a list of patients and associated general information or individual patient records, each patient record comprising a plurality of fields of healthcare-related data viewable, at the election of the provider, from the perspective of one or more of the group of aspects comprising demographics, enrollment data, medication, clinical information, notes, care plan and task list;
(d) means including the input means for entering into the memory means a new or modified patient record comprising health-related data collected during a patient interaction with the user, the record including at least demographic data, healthcare history data, appointment notes, clinical notes and care plan actions, the care plan actions including, at the user's option, the assigning of a provider to the patient based on data received during a patient interaction; and
(e) means responsive to selection of a care plan by a user for retrieving from the memory means and presenting on the display means guidelines and possible interventions related to the plan and for permitting the user to select particular care management plan for a patient from the various care plans, wherein said care management plan can be uniquely created by said user for said plurality of patients having at least one health condition.

2. The system according to claim 1, further including means for selecting care management plans in response to various information collected regarding one of the patients.

3. The system according to claim 2, further including means for devising a care management plan for at least one of said patients by allowing the user to select from various care plans, wherein said care management plan can be uniquely created by said user for said plurality of patients having at least one fitness and wellness goal.

4. The system according to claim 3, wherein said care management plans comprise categories selected by the user.

5. The system according to claim 4, wherein said categories comprise associated action items.

6. The system according to claim 5, wherein said action items comprise items selected from the group consisting of fitness and wellness programs, fitness and wellness goals, medications, testing and monitoring programs, informational programs, care plan goals, family and social support programs, disease specific organizations, signs and symptoms, self-monitoring programs and miscellaneous action items selected by user.

7. The system according to claim 6, wherein said miscellaneous action items are created to manage the healthcare plan of a patient, wherein said patient has unique health conditions that are not manageable by the selection of any existing miscellaneous action items.

8. The system according to claim 7, wherein said system permits the user to assign said action items to another user selected from the group consisting of physician, healthcare manager, and patient.

9. The system according to claim 8, wherein the patient is assigned the role of monitoring one or more action items selected by the user for her care plan.
